# EUROPEAN PATENT APPLICATION

(11) **EP 1 473 563 A2**
(43) Date of publication of application: **03.11.2004**
(21) Application number: 04252450.4
(22) Date of filing: 28.04.2004
(51) Int. Cl.: G01N 33/52

(54) **Test strip with clear base support layer for visual perception of a liquid sample during application**

(30) Priority: 29.04.2003 US 426457
(71) Applicant: LIFESCAN, INC., Milpitas, California 95035 (US)
(72) Inventor: Shartle, Robert J., Dorrington, CA 95223 (US); Matzinger, David P., Menlo Park, CA 94025 (US)
(74) Representative: Mercer, Christopher Paul

(57) **Abstract**

A test strip for use in the detection of an analyte (e.g., blood glucose) in a liquid sample (e.g., a whole blood sample) includes a base support layer and a reagent pad disposed on the base support layer. In addition, at least a portion of the base support layer in proximity to the reagent pad is formed of a clear material such that a user can perceive the liquid sample through the base support layer during application of the liquid sample to the reagent pad. The test strip optionally includes a reservoir for restraining excess liquid sample. The reservoir can be defined by hydrophilic and hydrophobic regions of the base support layer or by base support ridges that circumscribe the reagent pad. Also a test strip that includes a base support layer with a raised reagent pad platform, a reagent pad disposed on raised reagent pad platform and an upper layer disposed above the base support layer. At least a portion of the base support layer and at least a portion of the upper layer that are in proximity to the reagent pad are formed of a clear material such that a user can perceive the liquid sample through the base support and upper layers during application of the liquid sample to the reagent pad. In addition, the test strip is configured such that a capillary reservoir adapted for restraining excess fluid sample is defined by the base support layer and the upper layer.

## Description

### 1. Field of the Invention

The present invention relates, in general, to analytical test devices and, in particular, to test strips for the detection of an analyte.

### 2. Description of the Related Art

Various test strips have been developed for detecting the presence and/or the concentration of certain analytes in a liquid sample and/or chemical properties (e.g., pH or alkalinity) of a liquid sample. Such test strips can be used for the detection of, for example, glucose, cholesterol, proteins, ketones, phenylalanine or enzymes in a blood, urine or saliva sample. These test strips typically include one or more reagent pads (e.g., a reagent-impregnated membrane) that facilitate the detection of the analyte concentration or chemical property. For example, U.S. Patent No. 6,162,397 describes a visual blood glucose test strip with two reagent-impregnated membranes. Such reagent-impregnated membranes contain reagents that react with blood glucose to form visibly different colors. Further descriptions of conventional test strips and their reagent pads are available in U.S. Patent Nos. 4,900,666, 5,902,731 and 5,304,468.

Typical monitoring systems for the detection of an analyte in, or chemical property of, a liquid sample include a device, such as a portable hand-held meter, and a test strip to which the liquid sample (e.g., a whole blood sample) is applied. The device and test strip are used in tandem to detect an analyte concentration (e.g., blood glucose concentration) or other characteristic (e.g., prothrombin time and/or International Normalization Ratio [INR]) of the liquid sample. The device typically measures a property or properties of the test strip (e.g., an optical reflectance, optical transmittance or optical absorption) and then employs an algorithm(s) to calculate the characteristic based on the measured property or properties.

For the detection of blood glucose in a whole blood sample, a blood sample is most commonly expressed from a user's fingertip, where there is generally an abundant supply of blood due to the presence of capillary blood vessels, and then applied to the reagent pad of a test strip. Unfortunately, the nerve density in the fingertips can cause significant pain in many users. Sampling on alternative target sites, such as earlobes, palms, forearms and the abdomen, is sometimes practiced since these alternative target sites may be less sensitive. However, these alternative target sites generally have a less abundant supply of blood, which can result in liquid samples of relatively small volume.

Conventional test strips that are adapted for the application of small volume liquid samples are themselves often relatively small and, therefore, difficult for a user to manipulate. However, when a user must apply a liquid sample directly to a test strip that has not been inserted into a device (commonly referred to as "off-meter" test strip dosing), a relatively large test strip can obscure the user's view of the liquid sample. Furthermore, use of alternative target sites makes the application of a liquid sample to a test strip that has been previously and completely inserted into an associated device (commonly referred to as "on-meter" dosing) difficult. In addition, the application of inordinately large liquid samples to a test strip can result in excess liquid sample spreading beyond the reagent pad, creating an untidy condition on the test strip and leading to a subsequent contamination of an associated device (e.g., a hand-held meter).

Still needed in the field, therefore, is a test strip for the detection of an analyte in a liquid sample that is easy to manipulate and provides for user perception of the liquid sample during its application to the test strip. Also, the test strip should be simple to manufacture and prevent excess liquid sample from creating an untidy condition or contaminating an associated device.

### SUMMARY OF THE INVENTION

Test strips for the detection of an analyte in a liquid sample according to exemplary embodiments of the present invention are easy to manipulate and provide for a user to perceive a liquid sample (e.g., a whole blood sample that has been expressed from a target site) during application of the liquid sample to the test strip. Exemplary embodiments of test strips according to the present invention are also simple to manufacture and prevent excess liquid sample from creating an untidy condition or contaminating an associated device (e.g., a hand-held meter).

An exemplary embodiment of a test strip for use in the detection of an analyte (e.g., blood glucose) in a liquid sample (e.g., a whole blood sample) according to the present invention includes a base support layer and a reagent pad disposed on the base support layer. In addition, at least a portion of the base support layer in proximity to the reagent pad is formed of a clear material, thereby enabling a user to perceive the liquid sample through the base support layer during application of the liquid sample to the reagent pad. The test strip optionally includes a reservoir for restraining excess liquid sample. The reservoir can be defined, for example, by hydrophilic and hydrophobic regions of the base support layer or by ridges on the base support layer that circumscribe the reagent pad.

Another exemplary embodiment of a test strip for use in the detection of an analyte in a liquid sample according to the present invention includes a base support layer with a raised reagent pad platform, a reagent pad disposed on the raised reagent pad platform, and an upper layer disposed above the base support layer. At least a portion of the base support and upper layers that are in proximity to the reagent pad are formed of a clear material such that a user can perceive the liquid sample through the base support and upper layers during application of the liquid sample to the reagent pad. In addition, the test strip is configured such that the base support layer and the upper layer define a capillary reservoir therebetween that is adapted for restraining excess fluid sample.

Since exemplary embodiments of test strips according to the present invention are configured such that a user can perceive the liquid sample through the base support layer during application of the liquid sample to the reagent pad, the test strips are easily manipulated. In addition, since a user's view of the liquid sample is not obscured by the test strip itself, test strips according to the present invention can be of a relatively large and easily manipulated size. Furthermore, certain exemplary embodiments of the present invention include a reservoir that prevents excess liquid sample from creating an untidy condition or contaminating an associated device (e.g., a hand-held meter). Finally, test strips according to exemplary embodiments of the present invention include a minimal number of components and are, therefore, easily manufactured using, for example, conventional web-based and embossing techniques.

### BRIEF DESCRIPTION OF THE DRAWINGS

A better understanding of the features and advantages of the present invention will be obtained by reference to the following detailed description that sets forth illustrative embodiments, in which the principles of the invention are utilized, and the accompanying drawings, of which:
FIGs. 1A and 1B are simplified perspective and side views, respectively, of a test strip according to an exemplary embodiment of the present invention;
FIG. 2 is a simplified depiction of a manner by which a user can perceive a liquid sample through the test strip of FIGs. 1A and 1B;
FIGs. 3A and 3B are simplified top and side views, respectively, of a test strip according to another exemplary embodiment of the present invention;
FIGs. 4A and 4B are simplified top and side views, respectively, of a test strip according to yet another exemplary embodiment of the present invention;
FIGs. 5A and 5B are simplified top and side views, respectively, of a test strip according to yet another exemplary embodiment of the present invention;
FIG. 6 is a simplified top view of a test strip according to yet another exemplary embodiment of the present invention, wherein the base support layer includes a hydrophilic region adjacent to the reagent pad;
FIG. 7A and 7B are simplified top and cross-sectional side views, respectively of a test strip according to yet another exemplary embodiment of the present invention;
FIG. 8 is a simplified cross-sectional side view of a test strip according to yet another exemplary embodiment of the present invention, wherein the base support layer includes a raised reagent pad platform;
FIG. 9A is a perspective view of a test strip according to an exemplary embodiment of the present invention, wherein the test strip includes a reservoir defined by the test strip's bottom support layer, upper layer and spacer layer;
FIG. 9B is an exploded view of the test strip of FIG. 9A;
FIG. 9C is a simplified cross-sectional view of the test strip of FIG. 9A showing a liquid sample (LS) that has been applied to the test strip;
FIG. 9D is a simplified top view of the test strip of FIG. 9A with dashed lines indicating feature(s) that are blocked from view; and
FIG. 9E is a partial perspective cross-sectional view of the test strip of FIG. 9A.

### DETAILED DESCRIPTION OF THE INVENTION

To be consistent throughout the present specification and for clear understanding of the present invention, the following definitions are hereby provided for terms used therein:
The term "hydrophilic" refers to a surface property of a material that results in a contact angle of less than 90 degrees for an aqueous liquid sample of interest.
The term "hydrophobic" refers to a surface property of a material that results in a contact angle of 90 degrees or greater for an aqueous liquid sample of interest.
The term "highly wettable" refers to a surface property of a material that results in a contact angle of less than approximately 30 degrees for an aqueous liquid sample of interest.
The term "excess liquid sample" refers to a portion(s) of a liquid sample that has been applied to a test strip beyond (i.e., in excess of) the volume of the test strip's reagent pad or that has otherwise not been contained with the test strip's reagent pad.
The term "capillary channel" refers to a channel that, due to its geometry and surface properties, provides for movement of a liquid sample therethrough and/or therein via capillary action.

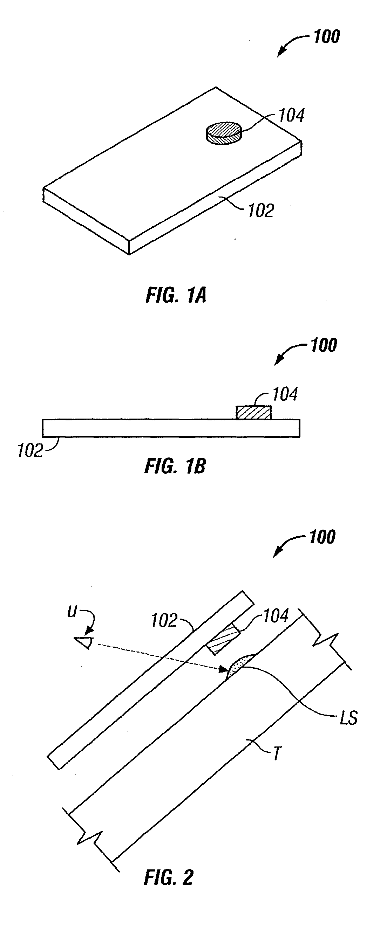

FIGs. 1A and 1B are simplified perspective and side views, respectively, of a test strip 100 for use in the detection of an analyte (e.g., blood glucose) in a liquid sample (e.g., a whole blood sample) according to an exemplary embodiment of the present invention. Test strip 100 includes a base support layer 102 and a reagent pad 104 (e.g., a reagent impregnated membrane) disposed on base support layer 102.

Base support layer 102 is configured such that at least a portion of the base support layer in proximity to reagent pad 104 is formed of a clear material, thereby providing for a user to perceive a liquid sample therethrough during application of the liquid sample to the reagent pad. However, for simplified discussion and easy understanding, it is assumed that base support layer 102 in the embodiment of FIGs. 1A and 1B is formed entirely of a clear material.

FIG. 2 is a simplified depiction (not to scale) of a manner by which a user can perceive a liquid sample by viewing the liquid sample through test strip 100. During application of a liquid sample (LS) to reagent pad 104, a user can, for example, hold test strip 100 such that reagent pad 104 is directed toward the liquid sample, as illustrated in FIG. 2. FIG. 2 depicts how a clear base support layer 102 will then provide a clear line of sight (represented by a dashed arrow in FIG. 2) between a user's point of view (U) and the liquid sample LS (e.g., a drop of whole blood) that has been expressed from a target site (T). The target site can be any suitable target site known to those skilled in the art, such as a fingertip or forearm target site, from which a liquid sample has been expressed.

As test strip 100 approaches liquid sample LS and target site T, the clear base support layer 102 enables a user to easily perceive the liquid sample, regardless of the dimensions of test strip 100. In addition, clear base support layer 102 can also enable a user to observe a liquid sample entering and filling the reagent pad. In this circumstance, a user can remove the test strip from the target site once the reagent pad has been filled by the liquid sample. Test strip 100 can, therefore, be of a relatively large and easily manipulated size without risk of obscuring a user's view of the liquid sample. Typical, but non-limiting, dimensions for the base support layer 102 of test strip 100 are a thickness in the range of approximately 0.003 to 0.025 inches, a width in the range of 0.25 to 0.60 inches and a length in the range of 1.1 to 2.25 inches.

Once the liquid sample has been applied to reagent pad 104 of test strip 100, test strip 100 can be either visually read (e.g., by comparison to a color chart) or inserted into an associated device (e.g., an associated hand-held meter) for an optical property measurement (e.g., an optical reflectance, optical transmittance or optical absorption). Such a visual reading or optical property measurement can be accomplished, if desired, through base support layer 102. In the circumstance that the reagent pad changes color in response to the application of a liquid sample, a clear base support layer can enable user to visually verify such a color change.

Optionally, base support layer 102 can be formed entirely of a clear material (as discussed above with respect to FIGs. 1A, 1B and 2) to provide for maximum manoeuvrability and ease of use. Alternatively, only a portion of base support layer 102 in proximity to reagent pad 104 need be formed of a clear material, as long as a user is able to adequately perceive a liquid sample during application of the liquid sample to the reagent pad. For example, base support layer 102 can be formed of a clear material for a distance of approximately 0.1 inch around a perimeter of the reagent pad and still provide a user with the ability to perceive a liquid sample during application of such to the reagent pad. If the base is clear, they can confirm that the membrane has change color from white to red, or whatever other color the membrane turns when sample is applied.

Referring again to FIGs. 1A and 1B, base support layer 102 can be formed of any suitable clear material known to those skilled in the art. Suitable clear materials include, but are not limited to, clear polyester materials, clear polycarbonate materials (e.g., 15 mil Makrofol DE polycarbonate material available from Tekra, New Berlin, Wisconsin, USA), clear acrylic materials, clear polystyrene materials, clear fluoropolymer materials and clear polyolefin materials.

In terms of a user's ability to easily manipulate test strips according to the present invention, it is particularly beneficial for the clear material from which the base support layer is formed to have a sufficient clarity to enable visual perception of a liquid sample (e.g., a drop of whole blood) through the base support layer at a distance of at least 0.5 inches. In choosing suitable clear materials, one skilled in the art can conduct a trial and error test to confirm sufficient clarity for a user's visual perception of a liquid sample through the base support layer during application of a liquid sample to the test strip's reagent pad.

To prevent excess liquid sample from spreading across the test strip, the clear material used to form base support layer 102 can be either inherently hydrophobic or treated in a manner which renders the base support layer hydrophobic. For example, a clear polyester base support layer can be siliconized using conventional and well known methods to render it hydrophobic. In the circumstance that a hydrophobic base support layer is employed, a liquid sample that comes into contact with the test strip will tend to either (i) flow into the reagent pad (rather than across the hydrophobic base support layer) or (ii) remain on the target site (e.g., a fingertip or forearm), thus preventing the liquid sample from creating untidy conditions on the test strip itself.

Reagent pad 104 can be any suitable reagent pad known in the art including, for example, a colorimetric reagent pad (e.g., a colorimetric reagent-impregnated membrane) for the detection of glucose in a whole blood sample. Suitable reagents for incorporation in a reagent pad include, for example, colorimetric reagents based on (i) a glucose oxidase (GO), horseradish peroxidase (HRPO), 3-methyl-2-benzothiazolinone hydrazone (MBTH) and dimethylamino benzoic acid (DMAB) chemistry; (ii) a GO, phenazine methosulfate (PMS), and 2,2'-Dibenzothiazolyl-5,5'-bis[4-di(2-sulfoethyl)carbamoylphenyl]-3,3'-(3,3'-dimethoxy-4,4'-biphyenylene)ditetrazolium disodium salt (available as WST-5 from Dojindo Molecular Technologies, Gathersburg, MD) chemistry and (iii) a glucose dehydrogenase (GDH), PMS and WST-5 chemistry. Although reagent pad 104 is illustrated as a single layer, it is envisioned that reagent pad 104 can be a single-layered or multi-layered reagent pad. Descriptions of suitable reagents and reagent pads are present in U.S. Patent Nos. 4,900,666, 5,304,468 and 6,420,128.

Suitable membranes for use as reagent pads include, for example, U.S. Filter BTS-30 membrane material (an asymmetrical polysolfone membrane), Pall Biodyne membrane material (a symmetrical Nylon membrane with a polyester inner support), and a Millipore Hi-Flow Plus membrane material (a membrane formed from nitrocellulose cast on a Mylar film).

As previously discussed, although base support layer 102 can be relatively large for easy manipulation, reagent pad 104 can still be relatively small and provide for the accurate detection of relatively small volume liquid samples (e.g., 0.5 to 1 microliter liquid samples). A typical thickness for the reagent pad is, for example, in the range of 0.001 inches to 0.010 inches, a typical diameter (for a circular reagent pad) is in the range of 0.030 inches to 0.25 inches. Reagent pad 104 can be affixed to base support layer 102 using any suitable means such as a clear pressure sensitive adhesive (e.g., a commercially available acrylic-based clear pressure sensitive adhesive such as those available from Adhesives Research, Inc., Glen Rock, Pennsylvania, USA) or a clear tape.

In the embodiment of FIGs. 1A, 1B and 2, base support layer 102 surrounds the entire perimeter of reagent pad 104. However, once apprised of the present disclosure, one skilled in the art will recognize that the base support layer of test strips according to the present invention need not surround the entire perimeter of the reagent pad in order to enable a user to perceive a liquid sample during application of such to the test strip's reagent pad.

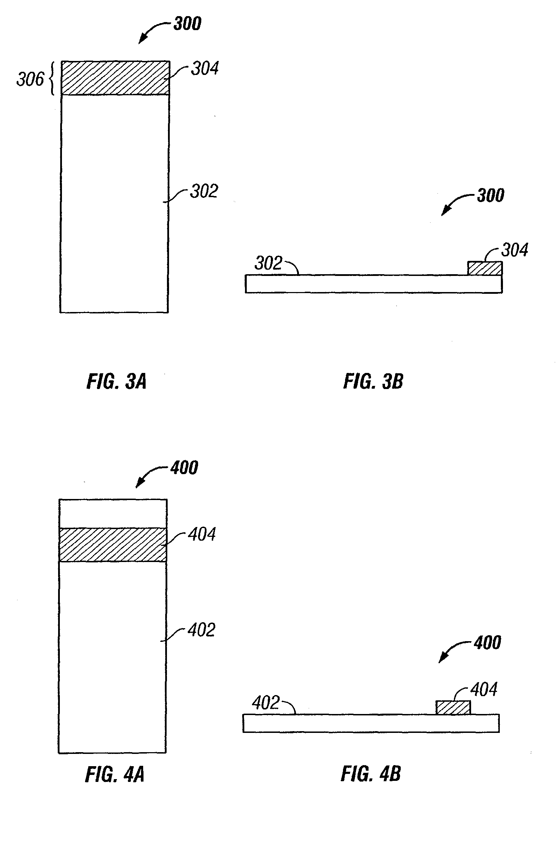

FIGs. 3A and 3B are simplified top and side views, respectively, of another test strip 300 for the detection of an analyte in a liquid sample according to the present invention. Test strip 300 includes a base support layer 302 and a reagent pad 304 disposed on an end 306 of base support layer 302. Base support layer 302 is configured such that at least a portion of the base support layer in proximity to the reagent pad (e.g., a portion within 0.1 inches of the reagent pad) is formed of a visually clear material, thereby enabling a user to perceive a liquid sample through the base support layer during application of such to the reagent pad. In the embodiment of FIGs. 3A and 3B, base support layer 302 surrounds only one side of reagent pad 304. The remaining sides of reagent pad 304 are open.

FIGs. 4A and 4B are simplified top and side views, respectively, of yet another test strip 400 for the detection of an analyte in a liquid sample according to the present invention. Test strip 400 includes a base support layer 402 and a reagent pad 404 disposed on the base support layer. Base support layer 402 is configured such that at least a portion of the base support layer in proximity to the reagent pad is formed of a visually clear material, thereby enabling a user to perceive a liquid sample through the base support layer during application of such to the reagent pad. In the embodiment of FIGs. 4A and 4B, base support layer 402 surrounds two sides of reagent pad 404. The remaining sides of reagent pad 404 are open.

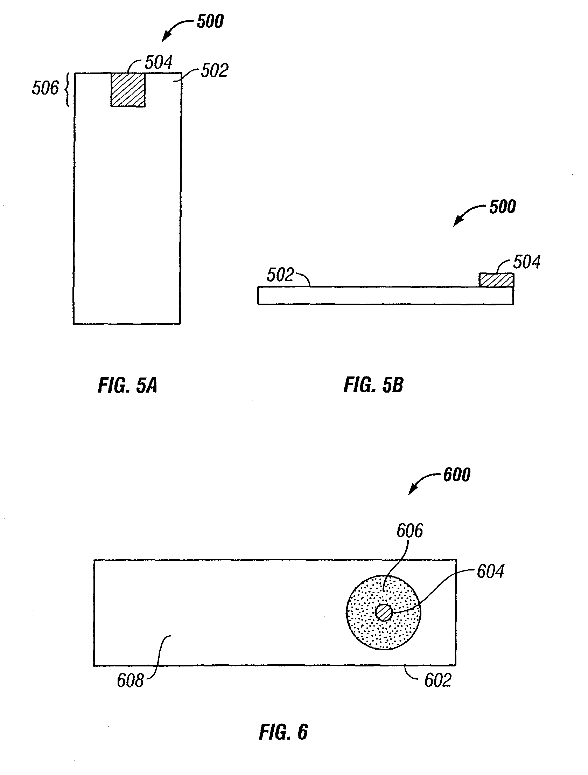

FIGs. 5A and 5B are simplified top and side views, respectively, of still another test strip 500 for the detection of an analyte in a liquid sample according to the present invention. Test strip 500 includes a base support layer 502 and a reagent pad 504 disposed on an end 506 of the base support layer. Base support layer 502 is configured such that at least a portion of the base support layer in proximity to the reagent pad is formed of a visually clear material, thereby enabling a user to perceive a liquid sample through the base support layer during application of such to the reagent pad. In the embodiment of FIGs. 5A and 5B, base support layer 502 surrounds three sides of reagent pad 504. The remaining side of reagent pad 504 is open.

In the embodiments of FIGs. 3A through 5B, the base support layer only partially surrounds the perimeter of the reagent pad. However, at least a portion of the base support layer in proximity to the reagent pad is formed of a visually clear material, thereby allowing a user to perceive a liquid sample through the base support layer during application of the liquid sample to the reagent pad. As described above, the base support layer can only be formed of a clear material for a distance of, for example, approximately 0.1 inch around the portion of the perimeter of the reagent pad adjacent to the base support layer, in order to adequately provide a user with the ability to perceive a liquid sample during application of such to the reagent pad.

To prevent excess liquid sample that has been applied to a test strip from contaminating an associated device (e.g., an associated hand-held meter), or otherwise creating an untidy condition, test strips according to various exemplary embodiments of the present invention include a reservoir for restraining the movement of excess liquid sample applied to the test strip. Such a reservoir can have any suitable volumetric capacity that is sufficient to restrain excess liquid sample. In the circumstance that the liquid sample is a whole blood sample expressed from a fingertip or alternate target site, the reservoir can have, for example, a volumetric capacity in the range of 5 microliters to 40 microliters.

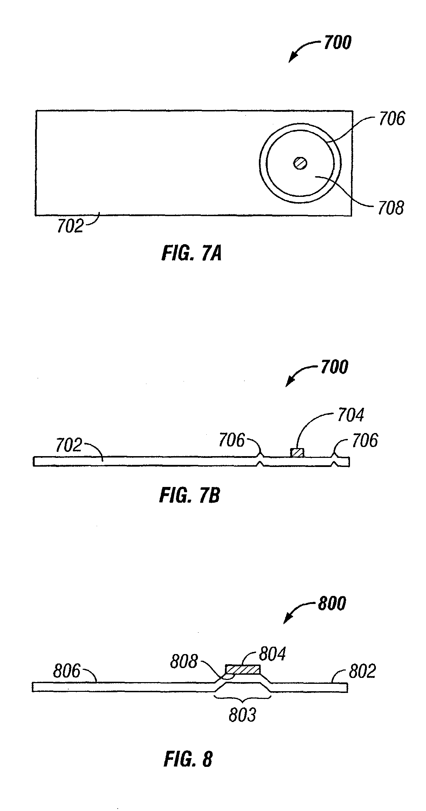

FIG. 6 is s simplified top view of a test strip 600 for detecting an analyte in a liquid sample according to an exemplary embodiment of the present invention. Test strip 600 includes a base support layer 602 and a reagent pad 604 disposed on the base support layer 602. Base support layer 602 is configured such that at least a portion of the base support layer in proximity to the reagent pad is formed of a visually clear material, thereby enabling a user to perceive a liquid sample through the base support layer during application of such to the reagent pad. Furthermore, base support layer 602 includes a hydrophilic region 606 disposed adjacent to reagent pad 604 and a hydrophobic region 608 spaced apart from the reagent pad by hydrophilic regions 606. In this embodiment, at least a portion of hydrophilic region 606 of base support layer 602 is visually clear.

In the embodiment of FIG. 6, hydrophilic region 606 serves as a reservoir for preventing the uncontrolled spread of a liquid sample across test strip 600. Excess liquid sample that is applied to reagent pad 604 is restrained within hydrophilic region 606 due to the inherent preference of the liquid sample to be disposed on hydrophilic region 606 rather than hydrophobic region 608. Hydrophilic region 606 and hydrophobic region 608 of base support layer 602 can be formed using any suitable technique known to one skilled in the art. For example, base support layer 602 can be initially formed from a hydrophobic material and hydrophilic region 606 subsequently formed thereon by the application of a layer of hydrophilic material, using conventional techniques (e.g., spraying or printing).

FIG. 7A and 7B are simplified top and cross-sectional side views, respectively, of yet another test strip 700 according to the present invention. Test strip 700 includes a base support layer 702 and a reagent pad 704 disposed on the base support layer. Base support layer 702 is configured such that at least a portion of the base support layer in proximity to the reagent pad is formed of a visually clear material, thereby enabling a user to perceive a liquid sample through the base support layer during application of such to the reagent pad.

Test strip 700 also includes a ridge 706 that circumscribes reagent pad 704. Circular ridge 706 is configured to prevent excess liquid sample from leaving the vicinity of reagent pad 704 and, thereby creating an untidy condition. Circular ridge 706, therefore, serves to define a reservoir between ridge 706 and reagent pad 704. One skilled in the art will appreciate that embodiments of test strips according to the present invention can optionally include additional ridges.

Dimensions for ridge 706 can be, for example, a height of 0.050 inches and a base width of 0.050 inches. Ridge 706 can be spaced apart from reagent pad 704 by a distance of, for example, 0.050 inches. Ridge 706 and/or the upper surface 708 of base support layer 702 that lies between ridge 706 and reagent pad 704 can be hydrophilic to enhance the reservoir's ability to restrain excess liquid sample. In this circumstance, the reservoir is essentially an open-faced capillary channel that will restrain excess liquid sample within the reservoir. Ridge 706 can be formed on base support layer 702 by any suitable technique known in the art including, for example, by embossing base support layer 702.

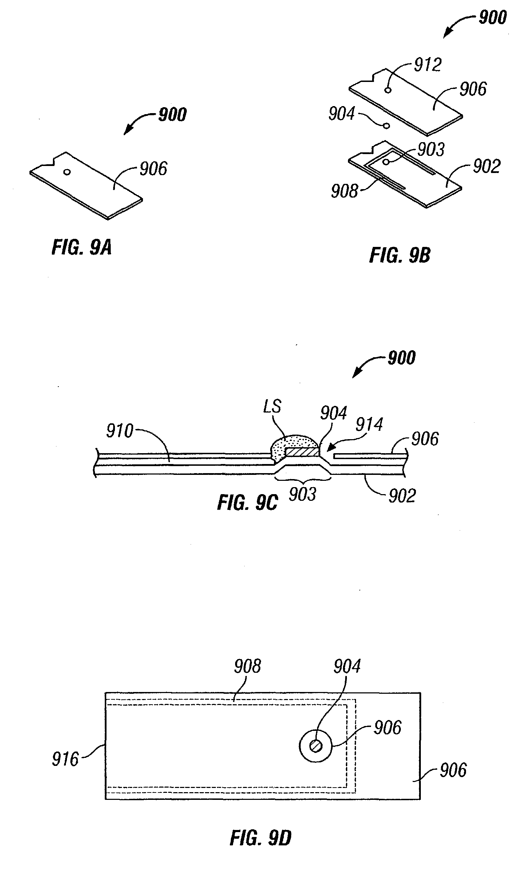

FIG. 8 is a simplified cross-sectional side view of yet another test strip 800 for the detection of an analyte in a liquid sample according to an exemplary embodiment of the present invention. Test strip 800 includes a base support layer 802 with a raised reagent pad platform 803 and a reagent pad 804 disposed on the raised reagent pad platform 803. Furthermore, base support layer 802 of test strip 800 is configured such that at least a portion of the base support layer in proximity to the reagent pad is formed of a visually clear material, thereby enabling a user to perceive a liquid sample through the base support layer during application of the liquid sample to the reagent pad.

Base support layer 802 has a major upper surface 806 and raised reagent pad platform 803 includes a platform upper surface 808 on which reagent pad 804 is disposed, as illustrated in FIG. 8. Platform upper surface can be spaced apart from (i.e., raised from) the major upper surface of the base support layer by, for example, a distance in the range of 0.020 inches to 0.20 inches, with a typical distance being 0.060 inches.

The disposition of reagent pad 804 on raised reagent pad platform 803 enhances the ability of a user to locate reagent pad 804 via the user's sense of touch by providing tactile feedback to the user. If desired, test strip 800 can include at least one ridge (not shown in FIG. 8), as described above with respect to test strip 700 (see FIGs. 7A and 7B), which defines a reservoir for restraining excess liquid sample. Raised reagent pad platform 803 can be formed on base support layer 802 using any suitable technique known to one skilled in the art including, but not limited to, embossing techniques.

Referring to FIGs. 9A-9E, another exemplary embodiment of a test strip 900 for detecting an analyte in a liquid sample (LS) according to the present invention includes a base support layer 902 with a raised reagent pad platform 903, a reagent pad 904 disposed on the raised reagent pad platform 903 and an upper layer 906 (see, in particular, FIG. 9C). Base support layer 902 also includes a raised spacer 908 that serves to define a capillary reservoir 910 for restraining excess liquid sample between base support layer 902 and upper layer 906. A typical height for the capillary reservoir is in the range of 0.001 inches to 0.010 inches.

Upper layer 906 includes an opening 912, through which raised reagent pad platform 903 and reagent pad 904 are exposed. The diameter of opening 912 is slightly larger than raised reagent pad platform 903, in order to create a gap 914 between reagent pad 904 and upper layer 906 through which excess liquid sample can be drawn into capillary reservoir 910. This feature of test strip 900 is illustrated in FIG. 9C, where a drop of liquid sample (LS) is shown on reagent pad 904 and entering capillary reservoir 910.

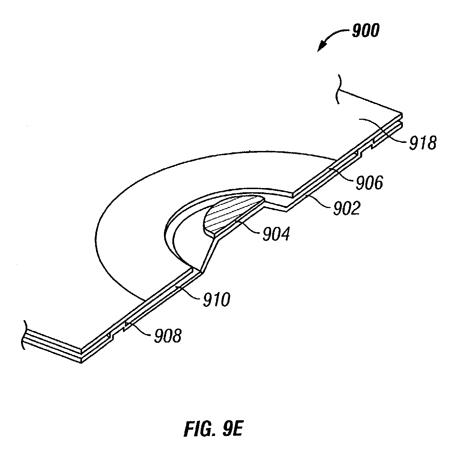

In the embodiment of FIGs. 9A-9E, one end of the capillary reservoir 910 is opened at an end of test strip 900 to provide a vent 916 (see, in particular, FIG. 9D). In addition, an opaque layer 918 (shown in FIG. 9E only) is disposed on top of upper layer 906 in order to obscure a user's view of any excess liquid sample that may have entered capillary reservoir 910. However, opaque layer 918 is spaced apart from reagent pad 904 such that a user can perceive a liquid sample through both base support layer 902 and upper layer 906 during application of the liquid sample to reagent pad 904. It should be noted, therefore, that at least a portion of both the base support layer and the upper layer that are in proximity to the reagent pad is formed of a clear material such that a user can perceive the liquid sample through the base support layer and upper layer during application of such to the reagent pad.

One skilled in the art will recognize that capillary reservoirs of test strips according to the present invention (such as capillary reservoir 910 of test strip 900) can be configured of various dimensions and defined in a variety of manners while still maintaining the ability to function as a capillary reservoir (i.e., to provide for the movement of a fluid sample from a gap surrounding a reagent pad into the capillary reservoir). To enable such movement of the liquid sample via capillary action, at least one of the surfaces defining the capillary reservoir should be hydrophilic in nature. The remaining surface(s) defining the capillary reservoir can be hydrophilic or hydrophobic, as long as the capillary reservoir provides for liquid movement via capillary action. In the embodiment of FIGs. 9A-9E, an upper surface of the base support layer (including any portions of the upper surface of the raised reagent pad platform that are not covered the reagent pad) and a lower surface of the upper layer portion that serve to define the capillary reservoir can both be equally hydrophilic nature, one of the surfaces can be more hydrophilic than the other surface, or one of the surfaces can be hydrophilic (e.g., highly wettable) while the other is hydrophobic.

Once apprised of the present disclosure, one skilled in the art will recognize that a capillary reservoir can be defined between a base support layer and an upper layer in test strips according to the present invention by means other than a raised spacer. For example, a spacer layer formed of double-sided adhesive tape can be employed to define a capillary reservoir therein.

It should be understood that various alternatives to the embodiments of the invention described herein may be employed in practicing the invention. It is intended that the following claims define the scope of the invention and that structures within the scope of these claims and their equivalents be covered thereby.

## Claims

1. A test strip for use in the detection of an analyte in a liquid sample, the test strip comprising:
a base support layer; and
a reagent pad disposed on the base support layer,
wherein at least a portion of the base support layer in proximity to the reagent pad is formed of a clear material such that a user can perceive the liquid sample through the base support layer during application of the liquid sample to the reagent pad.

2. The test strip of claim 1, wherein the portion of the base support layer formed of a clear material extends to a distance of at least 0.1 inches from the reagent pad.

3. The test strip of claim 1 or claim 2, wherein at least one surface of the base support layer is hydrophobic.

4. The test strip of any one of claims 1 to 3, wherein the base support layer surrounds an entire perimeter of the reagent pad.

5. The test strip of any one of claims 1 to 3, wherein the base support layer partially surrounds a perimeter of the reagent pad.

6. The test strip of any one of claims 1 to 5, wherein the base support layer includes a raised reagent pad platform and the reagent pad is disposed on the raised reagent pad platform.

7. The test strip of claim 6, wherein the base support layer has a major upper surface and the raised reagent pad platform includes a platform upper surface, the platform upper surface being spaced from the major upper surface of the base support layer by a distance in the range 0.02 inches to 0.2 inches.

8. The test strip of any one of claims 1 to 7, further comprising a reservoir for restraining excess liquid sample applied to the test strip.

9. The test strip of claim 8, wherein the reservoir is a capillary reservoir.

10. The test strip of claim 9, wherein the reservoir is an open-faced capillary channel.

11. The test strip of any one of claims 8 to 10, wherein the reservoir has a volumetric capacity in the range of 5 microliters to 40 microliters.

12. The test strip of any one of claims 8 to 11, wherein the base support layer includes a hydrophilic region adjacent to the reagent pad and wherein the hydrophilic region serves to define the reservoir.

13. The test strip of any one of claims 8 to 11, further including at least one ridge on the upper surface of the base support layer, the ridge at least partially circumscribing the reagent pad and wherein the ridge serves to define the reservoir.

14. The test strip of claim 13, wherein the ridge circumscribes the reagent pad.

15. The test strip of any one of claims 8 to 11, further including an upper layer disposed above the base support layer, and wherein the reservoir is a capillary reservoir between the upper layer and the base support layer.

16. The test strip of claim 15, wherein the reservoir is defined by at least one ridge disposed on the base support layer, the ridge being spaced apart from the reagent pad.
